# EUROPEAN PATENT SPECIFICATION

(11) **EP 3 265 437 B1**
(45) Date of publication and mention of the grant of the patent: **05.12.2018**
(21) Application number: 16721965.8
(22) Date of filing: 04.03.2016
(51) Int. Cl.: C07C 5/333, C07C 7/144, C07C 11/06, C07C 11/04, C07C 11/08, C07C 15/46

(54) **SYSTEM AND METHOD FOR THE PRODUCTION OF ALKENES BY THE DEHYDROGENATION OF ALKANES**
SYSTEM UND VERFAHREN ZUR HERSTELLUNG VON ALKENEN DURCH DEHYDRIERUNG VON ALKANEN
SYSTÈME ET PROCÉDÉ POUR LA PRODUCTION D'ALCÈNES PAR DÉSHYDROGÉNATION D'ALCANES

(30) Priority: 05.03.2015 EP 15157764
(43) Date of publication of application: 10.01.2018
(73) Proprietor: Stamicarbon B.V. acting under the name of MT Innovation Center, 6135 KW Sittard (NL)
(72) Inventor: CROPPI, Chiara, I-00148 Rome (IT); MARTORELLI, Francesco Simone, I-00148 Rome (IT); PALO, Emma, I-00148 Rome (IT); IAQUANIELLO, Gaetano, I-00148 Rome (IT); SALLADINI, Annarita, I-00148 Rome (IT)
(74) Representative: V.O.
(86) International application number: PCT/NL2016/050150
(87) International publication number: WO 2016/140574

(56) References cited:
- WO-A1-2012/134284
- GB-A- 2 201 159

## Description

### Field of the Invention

The invention is in the field of the production of alkenes (olefins) by the catalytic dehydrogenation of corresponding alkanes. The invention particularly pertains to the production of propene by the selective dehydrogenation of propane. The invention provides a method and a plant.

### Background of the invention

Olefinic compounds (alkenes) are widely used in a number of chemical industries. To name a few, for the production of petrochemical products, such as synthetic rubbers, plastics, motor fuel blending additives. Among the olefins, propylene (propene) is the world's second largest petrochemical commodity, being the precursor of polypropylene, which is used in such everyday products as packaging materials and outdoor clothing.

Catalytic dehydrogenation of alkanes is becoming a growing branch in petrochemical industry as a route to obtain alkenes from low-cost feedstocks of saturated hydrocarbons (alkanes), according to the reaction equation (1):

CₙH₂ₙ₊₂ ↔ CₙH₂ₙ + H₂ (1)

As compared to conventional cracking technologies, catalytic dehydrogenation may provide better selectivity at lower temperatures, lowering also the coke deposition rate. However, an issue is the deposition of coke on the catalyst, as well as a reduction of the active specific surface area as a result of catalyst particle agglomeration (sintering).

An approach to overcome the limitations of the dehydrogenation of alkanes is represented by the use of membrane reactors, in which the chemical reaction is coupled with the separation of one of the end products, such as hydrogen. Whilst this has advantages in terms of, *inter alia*, conversion and milder operating conditions, coke deposition is still a problem.

A background reference addressing the foregoing, is WO 2012/134284. Therein a process and a plant are provided allowing the catalytic dehydrogenation of alkanes to occur with a higher alkane conversion, yet without a similar promotion of coke formation. The process and plant described are referred to as an "open architecture."

As is known to the skilled person, membrane reactor configurations are of the "open architecture" or "closed architecture" type. See, e.g., Angelo Basile, Ed., Handbook of Membrane Reactors (2013); Volume 2: Reactor Types and Industrial Applications, pages 469-471.

In a membrane reactor configuration according to the closed architecture, the separation membrane is integrated in a reactor. Typically, such a reactor is composed of two concentric tubes, where a catalyst is packed in the annular zone (between the concentric tubes) and the inner tube is a membrane. The closed architecture can also be arranged in the reverse order, i.e., having the catalyst in an inner tube, the membrane layer at the internal surface of said inner tube, and thus the permeate side in the annulus, outwards from said inner tube. In the event of hydrocarbon conversion, a feed (e.g. propane) enters the zone where the catalyst is placed (such as the annular zone as discussed above). There it is converted to desired product (e.g. propene in the event of a propane feed) and whereby hydrogen is separated through the membrane. Hydrogen is preferably removed with the aid of a sweeping gas.

An open architecture refers to a configuration wherein a reactor is not itself a membrane reactor, but wherein outside of the reactor, and downstream thereof, a selective membrane is placed. In the open architecture, another reaction unit is needed downstream of the membrane separation module. After the membrane separation, separated hydrogen is removed with sweep gas, and a retentate (converted hydrocarbon) is sent to said further reactor. Generally, this set of units is repeated.

Whilst the closed architecture has the advantage of being more compact, the open architecture provides for more flexibility in operation of the process. Particularly, the open architecture allows the decoupling of reaction and separation unit operating conditions, meaning that the temperatures in either unit can be optimized independently of each other. Thereby one has to accept a loss of compactness, a larger membrane surface, and higher costs.

The invention seeks to provide a process and equipment for the membrane-assisted catalytic dehydrogenation of hydrocarbons, whereby product yields can be obtained that are better still than achievable in conventional closed architecture plants, whilst retaining the process optimization achievable in accordance with an open architecture configuration.

### Summary of the Invention

In order to better address the foregoing desires, the invention presents, in one aspect, a method for the production of an alkene by the dehydrogenation of a corresponding alkane, comprising the steps of:
(i) providing a hydrocarbon source comprising at least one alkane;
(ii) subjecting, in a first reactor system, the hydrocarbon source to a first dehydrogenation reaction in the presence of a dehydrogenation catalyst, so as to form a first reaction mixture comprising hydrogen, unreacted alkane, and an initial yield of the alkene corresponding to said at least one alkane;
(iii) in a first separation step, subjecting the reaction mixture to membrane separation so as to obtain a permeate comprising hydrogen and a retentate comprising an alkene-enriched reaction mixture;
(iv) feeding said alkene enriched reaction mixture to a second reactor system, wherein unreacted alkane comprised in said reaction mixture is subjected to a second dehydrogenation reaction in the presence of a dehydrogenation catalyst so as to form a second reaction mixture comprising hydrogen and a further yield of the alkene corresponding to said at least one alkane;
(v) in a second separation step subjecting the second reaction mixture to membrane separation so as to remove hydrogen, thereby producing a further alkene-enriched reaction mixture;
wherein the first dehydrogenation reaction and the first separation step are conducted in separate reaction and separation units, and the second dehydrogenation reaction and the second separation step are conducted in at least one integrated reaction and separation unit.

In another aspect, the invention provides a plant for the production of an alkene by the dehydrogenation of a corresponding alkane, said plant comprising a first reactor for conducting a catalytic dehydrogenation reaction, downstream of said first reactor, and in fluid communication therewith, a first membrane separator for separating hydrogen from a dehydrogenation reaction mixture, and downstream of said first membrane separator, and in fluid communication therewith, a second reactor, wherein said first reactor and separator are constructed as separate units, and wherein the second reactor comprises a second membrane separator, said second reactor and separator being constructed as a single unit.

In yet another aspect, the invention includes a method of modifying an existing olefin production plant comprising at least one membrane-assisted dehydrogenation reactor having a configuration according to closed architecture, by placing a membrane-assisted dehydrogenation reactor system having a reactor and membrane configuration as applicable to an open architecture configuration upstream of the at least one existing reactor.

In a still further aspect, the invention is a method of modifying an existing olefin production plant comprising at least one membrane-assisted dehydrogenation reactor system having a configuration according to open architecture, comprising a reactor unit and downstream of said reactor unit, a membrane separation unit, the method comprising adding to the plant a membrane-assisted dehydrogenation reactor having a configuration in accordance with closed architecture, downstream of the membrane separation unit of the at least one existing reactor system.

### Brief description of the drawings

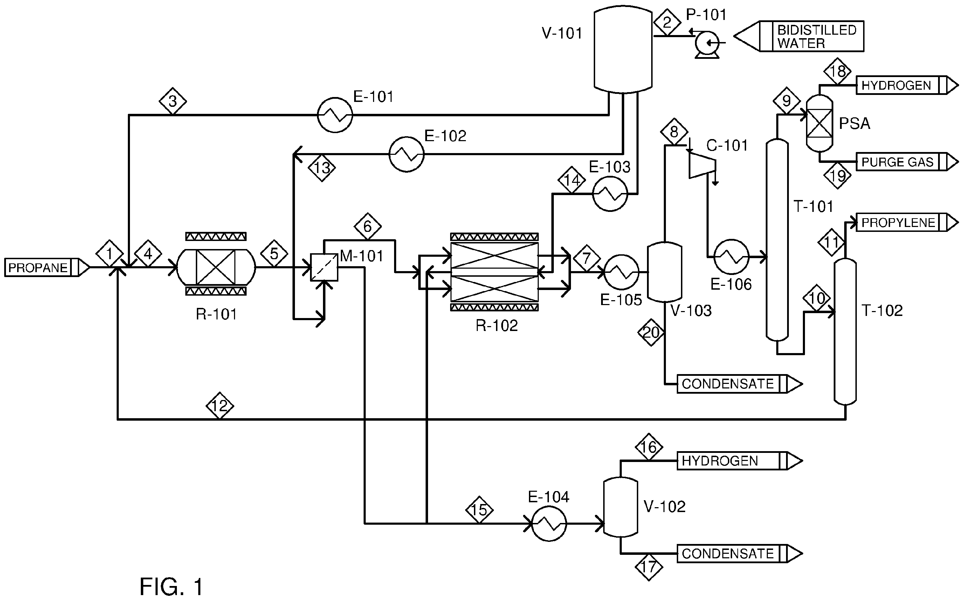

Fig. 1 depicts a process scheme of a hybrid plant configuration according to the invention.

### Detailed description of the invention

In a broad sense, the invention provides a hybrid reactor configuration, specifically having an upstream membrane reactor configuration according to open architecture and a downstream membrane reactor configuration of closed architecture. Thereby the term "closed architecture" indicates a membrane reactor configuration wherein a separation membrane is integrated in a reactor. The term "open architecture" indicates a membrane reactor configuration comprising a set of at least two reactors that are not themselves membrane reactors, but wherein outside of the reactors, downstream of a first reactor and upstream of a second reactor, and in fluid communication with both reactors, a membrane separation unit is positioned.

In the present description, a reactor indicates a reactor suitable for conducting a catalytic dehydrogenation reaction. The general features of such reactors are known to the skilled person and do not require elucidation here. Some of these features are the regular inlets and outlets for, respectively, gaseous or liquefied feedstocks and obtained reaction mixtures. In an interesting embodiment, the alkane (such as propane) is fed to the reactor in a gaseous phase, and is available at battery limits in a gaseous phase. In another interesting embodiment, e.g. when working at increased pressure, the alkane (such as propane) is stored in a liquid phase, pumped, vaporized, and fed to the reactor. Other such features include provisions for adjusting pressure and temperature. It will be understood that a reactor for catalytic dehydrogenation will be equipped with an arrangement for the presence of a dehydrogenation catalyst.

The placing of an open architecture membrane reactor system upstream of a closed architecture membrane reactor system brings advantages to the operation of both reactor systems. In membrane reactors of closed architecture, the membrane is frequently present over the full length of the reactor. As a result, an upstream part of the membrane will inevitably not be used efficiently, since at this point the reaction has not yet proceeded sufficiently far to substantially form hydrogen. Without hydrogen to be removed, the membrane is basically without function. Since these separation membranes are expensive, it would be desired to make more efficient use of the entire membrane also in a membrane reactor of closed architecture. Interestingly, the removal of hydrogen from the reaction mixture downstream of the dehydrogenation reactor by definition is never complete. Accordingly, there is always a small amount of hydrogen that is fed to the successive stage of reaction.

In the set-up according to WO 2012/134284, such remaining hydrogen will be carried to the next reactor. Thus, in the event of a sequence of membrane reactor systems of open configuration, from each subsequent system additional remaining hydrogen will be included in the reaction mixture. This may lead to an increasingly inefficient removal of hydrogen. The placing of a closed architecture membrane reactor downstream of an open architecture membrane reactor system, thus makes that hydrogen not removed in the open architecture, will enter the closed architecture membrane reactor at the initial, otherwise non-used portion of the separation membrane in said reactor. This provides another stage of hydrogen removal, thereby in total more efficiently removing hydrogen from the reaction mixture. Moreover, this means that also the initial portion of the separation membrane in the closed architecture-based reactor, is now put to advantageous use.

Further, the hybrid architecture of the present invention serves to maximize the yield in alkene production, while limiting the catalyst deactivation rate. Without wishing to be bound by theory, the inventors believe that this can be attributed in particular to the fact that the second (integrated membrane) reactor is provided with a feed that contains hydrogen retained from the previous stage. A continuous presence of hydrogen in the reaction environment aids in the reduction of coke formation on the catalyst. The hydrogen results from the aforementioned incomplete removal by the membrane separator upstream of the second reactor.

It will be understood that the hydrogen removal upstream of the second reactor should be of a sufficient order of magnitude to ensure that the chemical equilibrium in said second reactor is adequately shifted towards further dehydrogenation. Generally, at least 50% of the hydrogen obtained in the first reactor will be removed prior to the entry of the reaction stream of the first reactor, as a feed to the second reactor.

For completeness' sake, it is added that the terms "first and second" reactor are relative. More reactors can be present, but at any rate the invention is based on a configuration wherein part of an open architecture configuration (viz., a non-membrane reactor and, downstream thereof and in fluid communication therewith, a membrane separator) is directly connected to a membrane-reactor of closed architecture (i.e., one having an integrated membrane separator). Herein the (open architecture) non-membrane reactor is the "first" reactor and the downstream closed architecture membrane reactor is the "second" reactor.

The invention is applicable to both a process and a plant. The plant used for carrying out the process comprises a first reactor for conducting a catalytic dehydrogenation reaction. Downstream of said first reactor, the plant comprises a first membrane separator for separating hydrogen from a dehydrogenation reaction mixture. This constitutes a first membrane-assisted reactor system of open architecture. Downstream of said first membrane separator, the plant comprises a second reactor. The second reactor comprises a second membrane separator, said second reactor and separator being constructed as a single unit in accordance with a closed architecture. It will be understood that the various reactors and units are in fluid communication with each other so as to be able to conduct the process of the invention. Thereby a gas outlet of the first membrane separator is in fluid communication with a gas inlet of the second reactor. Other inlets and outlets for feedstock, products, and by-products are provided for in accordance with normal practice in the art.

In an interesting embodiment, the plant of the invention comprises the aforementioned hybrid configuration more than one time. This can be either in series or parallel. Preferably, the plant comprises 3-5 hybrid reactor configurations. Placed in series, this refers to, in downstream order:
i) a first non-membrane reactor;
ii) a first membrane separation unit;
iii) a first membrane-assisted reactor of closed configuration;
iv) a second non-membrane reactor;
v) a second membrane separation unit;
vi) a second membrane-assisted reactor of closed configuration;
vii) a third non-membrane reactor;
viii) a third membrane separation unit;
ix) a third membrane-assisted reactor of closed configuration
   (and possibly continuing in similar manner).

In an alternative embodiment, a plurality of reactors can be placed in parallel. Such a parallel configuration can be considered in view of the advantage that a plant can continue to operate via one line, whilst, at the same time, in a parallel line maintenance is conducted such as catalyst decoking and activation. In such a configuration, the plant of the invention will have at least one line in accordance with the hybrid set-up of an upstream part of an open architecture configuration (i.e. non-membrane reactor and a membrane separator as discussed above), and a downstream membrane-assisted reactor in accordance with closed architecture. Preferably, more parallel lines will have the hybrid configuration of the invention.

The invention not only provides process advantages, but is also beneficial in respect of modifying pre-existing plants. WO 2012/134284 discloses a method of turning conventional olefin production plants into membrane-assisted plants. The present invention, on the other hand, allows a further improvement of existing membrane-assisted plants.

As mentioned above, such plants are either of the "open architecture" type or, as many disclosed membrane plant configurations, of the "closed architecture" type. The present invention can be applied to modifying either type of plant.

In the event of an open architecture plant, a modifying step in accordance with the invention will be to insert a membrane-assisted dehydrogenation reactor of closed architecture directly downstream of a membrane separation unit present in the plant as part of a membrane-assisted dehydrogenation reactor system of open architecture. Hereby "directly downstream" refers to the fact that a gas inlet of the inserted reactor is in fluid communication with a gas outlet of the membrane separation unit without the gas being led through a unit wherein it would be chemically altered. This is generally by means of ducts or gas-flow lines, possibly including one or more valves, pumps, or reservoirs.

It is noted that existing plants of open architecture will normally comprise a second reactor downstream of a first membrane separation unit. This second reactor can itself be part of a second membrane-assisted reactor of open architecture. In this event, one can optionally replace said second reactor of open architecture by the added reactor of closed architecture. One can optionally also just insert the added reactor between two pre-existing open-architecture membrane reactor configurations.

The invention is also applicable to modernizing a pre-existing membrane-assisted catalytic dehydrogenation plant based on closed architecture. Here the invention comprises adding a membrane-assisted dehydrogenation reactor system of open architecture directly upstream of an already present membrane reactor of closed configuration. Accordingly, one adds a reactor for catalytic dehydrogenation operating without a membrane, and a membrane separation unit downstream of said reactor and in fluid communication therewith. The reactor system will be added so as to have a gas outlet of the membrane separation unit in fluid communication with a gas inlet of the pre-existing closed architecture membrane reactor.

It will be understood that the invention is also applicable in the event that either type of pre-existing plant comprises a plurality of reactors (in parallel or in series). The modification by adding a reactor of the appropriate type can then be done in relation to one or more of the already present reactors, as desired.

It will be understood, that the infrastructure of the plant, e.g. energy supply lines, gas flow lines, control systems, will normally require to be upgraded in order to accommodate the operation of the additional units. This is well within the ambit of the skilled persons regular skills.

The method of the invention can be performed on a wide variety of hydrocarbon sources comprising one or more alkanes. This generally refers to any fossil fuel rich mixture. Under fossil fuel it is understood here carbon containing natural fuel material and preferably gas material such as natural gas, methane, ethane, propane, butane and mixtures thereof. In an interesting embodiment ethyl benzene is used, so as to yield styrene. Preferably, light hydrocarbons (preferably C₂-C₄) are used in the dehydrogenation reaction according to the invention, with ethane and, particularly, propane being most preferred. Nevertheless, in general, the invention is applicable to all alkanes that can be subject to catalytic dehydrogenation. This wide choice of alkanes is known to the skilled person. Suitable alkanes, e.g., are straight-chain or branched alkanes having chain lengths of 2 to 20 carbon atoms. Preferably, the invention is employed on C₂-C₁₀ alkanes, and more preferably on C₂-C₆ alkanes. Most preferably, the invention is used in the production of light olefins (C₂-C₄), such as ethylene, propylene, or isobutene, starting from the corresponding (C₂-C₄) alkanes.

In all instances, the process can be operated on starting materials that either provide a mixture of alkanes, or a specific isolated alkane. The starting materials can be purified or crude.

Suitable dehydrogenation catalysts, and methods of conducting the catalytic dehydrogenation reaction, are known in the art. Thus the process conditions for catalytic dehydrogenation are well known to a person skilled in the art. Reference is made, e.g., to "Chemical Process Technology" by J.A. Moulijn, M. Makkee, A. van Diepen (2001) Wiley.

Generally, before entering the dehydrogenation environment, an alkane rich mixture is compressed (e.g. in the case of a propane-rich gas mixture) up to 5-10 barg and preheated, e.g. in a charge heater, to the reaction temperature, and directed to the dehydrogenation reactor at an atmospheric or sub-atmospheric pressure. Generally, the catalytic dehydrogenation reaction takes place at temperatures ranging between 550-700°C and at sub-atmospheric pressure, preferably 0.5-0.7 atm (0.05 to 0.07 MPa) or slightly above. Typical dehydrogenation catalysts contain platinum or chromium. In a preferred embodiment Cr based catalysts deposited on Al₂O₃ are used.
In the state of the art, the alkane (e.g. propane) frequently is fed at atmospheric or sub-atmospheric pressure. In the process of the invention it is preferred to feed compressed alkane, since the membrane separation is favored by high partial pressure difference between retentate and permeate side.

After the dehydrogenation reaction, the resulting reaction mixture (e.g. a gas mixture comprising propylene and hydrogen) is carried to a membrane separator, typically based on palladium or palladium alloy, to separate the hydrogen. Membranes for separation of hydrogen are known. Generally, these can be polymeric membranes or metal membranes. Metal membranes are preferred, with palladium or palladium alloys such as for example Pd-Ag being the most preferred. Most preferred are thin palladium membranes, typically having a thickness of the order of micrometers in size, preferably having a thickness of from 1 µm to 3 µm. The use of thin membranes has the advantaged of helping to increase the hydrogen flux.

In the invention, it is preferred to employ metallic rather than polymeric membranes. This is of advantage, since the higher stability of metallic membranes, as compared to polymeric membranes, allows the hydrogen separation to be conducted at a temperature of the same order of magnitude, and preferably just the same temperature, as the temperature at the reactor outlet. The use of polymeric membranes would require cooling to a temperature below 300°C. Particularly in the event that a plurality of reactor/separator units (open architecture) and membrane reactors (closed architecture) are employed in line, it is advantageous to avoid cooling, since the next reactor unit will desirably operate at a reaction temperature of the original order of magnitude. Hence, the lower the temperature at the separation units, the higher the temperature difference that needs to be overcome until the desired reaction temperature is reached.

The invention is explained further with reference to the scheme in Fig.1. The figure is not limiting, but serves to illustrate an embodiment of the invention. E.g., in the description of the figure, reference is made to the dehydrogenation of propane, but the described configuration will be generally applicable also to the dehydrogenation of other alkanes, such as methane, ethane or butane.

A mixture of propane and steam is fed to a first not integrated reactor where a catalyst (e.g. Pt based) is loaded. The amount of steam in the first stage is preferably 20% but it could be changed in the range 5-60% in order to balance the negative effect of hydrogen removal in integrated reactor on coke formation. Some additional steam feed could be also foreseen only in the integrated reactor.

The operating pressure is 5.4 bar in order to operate directly with gaseous propane, but the plant could be operated in the range up to 20 bar. Of course in this case a vaporizing system for the feed propane is necessary. Too high operating pressure, even if positive for hydrogen permeation across the membrane, could be detrimental for the reaction stage, since the reaction is thermodynamically affected by high pressure.

The operating temperature of the reactor for propane is preferably about 500°C but a suitable range is, e.g., between 450 and 540°C. The lower limit is generally linked to the threshold temperature of the catalyst, whereas the upper limit is linked to the temperature of the heating fluid. In the case of molten salts it is preferred to work below 550°C in order to avoid molten salts deterioration. It will be understood that the choice of heating fluid is not limited to the use of molten salts. E.g. also the exhaust from a gas turbine can be employed. The partially converted stream is further routed to the first membrane separator.

On the permeate side, e.g. steam is used as sweep gas, to further reduce the hydrogen partial pressure on permeate side, thus increasing the hydrogen flow across the membrane. The use of steam as sweep gas is preferred in order to easily separate the hydrogen just by condensation. It will be understood that other gases, such as nitrogen, can also be employed.

The retentate from the separator is routed to the integrated membrane reactor. The reactor is preferably operated at 450-540°C, and most preferably at about 500°C in order to further reduce coke formation on the catalyst.

In an interesting embodiment, the product stream obtained from the dehydrogenation process in the hybrid membrane-assisted reactor configuration of the invention, is separated into an alkene stream (such as propene) and a stream of unreacted alkane (such as propane). The latter stream is advantageously recycled, preferably as a feed to the first reactor. In this respect, the process of the invention, in any embodiment, preferably comprises a step wherein unreacted alkane retrieved from the second reactor system is recycled to the first reactor system. It will be understood that, to this end, the plant used for carrying out the process of the invention, preferably comprises the appropriate flow lines, such as tubing, enabling the recirculation of unconverted alkane as foreseen. It is also possible to include a recirculation loop from the downstream end of the first reactor system, but upstream of the second reactor system, back to the upstream end of the first reactor system. This will allow recirculating part of the unreacted alkane from the first reactor system, whilst another part of the unreacted alkane will be led, in accordance with the invention, to the second reactor system.

E.g., in Fig. 1 the retentate steam (7) is cooled (E-105), the condensate is separated (V-103) and the gas stream (8) is compressed (C-101). After another cooling step (E-106) is sent to a deethanizer column (T-101) which produces a lighter fraction or offgas (9) rich in ethane , that is sent to a PSA unit in order to recover hydrogen, and an heavier fraction (10) comprising propylene and unreacted propane. This is sent to a further separation column (T-102) from which a propylene stream (11) and a propane stream (12) are obtained. The propane stream (12) is recycled and mixed with fresh propane. The full legend of Fig. 1 is as follows:

### Equipment:

R-101 not-integrated (i.e.: non-membrane) reactor
M-101 membrane separator
R-102 integrated membrane reactor
V-103 condensate separator
T-101 deethanizer
T-102 splitter propane/propylene
V-102 condensate separator
E-101, E-102, E-103 steam generator and superheater
E-104, E-105, E-106 cooler
C-101 dry retentate compressor
V-101 bidistilled water tank
P-101 bidistilled water pump

### Streams:

1 fresh propane;
2 bidistilled water;
3 process steam;
4 feed to the not-integrated reactor;
5 effluent of not-integrated reactor;
6 retentate from membrane separator;
7 retentate from integrated membrane reactor;
8 dry retentate;
9 top of deethanizer column;
10 bottom of deethanizer column;
11 propylene, stream;
12 recycle propane;
13 sweep gas to membrane separator;
14 sweep gas to integrated membrane reactor;
15 permeate from membrane separator and integrated membrane reactor;
16 hydrogen from permeate;
17 condensate from permeate;
18 hydrogen from PSA;
19 purge gas from PSA;
20 condensate from retentate

While the invention has been illustrated and described in detail in the drawings and foregoing description, such illustration and description are to be considered illustrative or exemplary and not restrictive; the invention is not limited to the disclosed embodiments

For example, it is possible to operate the invention in an embodiment wherein part of an existing plant having a plurality of catalytic dehydrogenation reactors is modified in accordance with the invention, and another part is not modified. Also, the other part can be modified in accordance with the disclosure in WO 2012/134284. In an alternative embodiment, the reactant stream from the first (open architecture) reactor is led, wholly or partly, directly to the second (closed architecture) reactor, whilst bypassing the first (open architecture) membrane separator.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum the invention relates to a method and plant for the catalytic dehydrogenation of alkanes, such as propane. The plant is of hybrid architecture wherein one or more membrane-assisted reactor configurations according to open architecture are combined with one or more membrane-containing reactors of closed architecture. Hydrogen remaining in the reaction mixture after separation in the membrane separation unit of a first open architecture configuration, is fed to a first membrane-reactor of the closed architecture type. Also part of the invention are methods of modifying plants so as to create the hybrid architecture plant.

## Claims

1. A method for the production of an alkene by the dehydrogenation of a corresponding alkane, comprising the steps of:
(i) providing a hydrocarbon source comprising at least one alkane;
(ii) subjecting, in a first reactor system, the hydrocarbon source to a first dehydrogenation reaction in the presence of a dehydrogenation catalyst, so as to form a first reaction mixture comprising hydrogen, unreacted alkane, and an initial yield of the alkene corresponding to said at least one alkane;
(iii) in a first separation step, subjecting the reaction mixture to membrane separation so as to obtain a permeate comprising hydrogen and a retentate comprising an alkene-enriched reaction mixture;
(iv) feeding said alkene enriched reaction mixture to a second reactor system, wherein unreacted alkane comprised in said reaction mixture is subjected to a second dehydrogenation reaction in the presence of a dehydrogenation catalyst so as to form a second reaction mixture comprising hydrogen and a further yield of the alkene corresponding to said at least one alkane;
(v) in a second separation step subjecting the second reaction mixture to membrane separation so as to remove hydrogen, thereby producing a further alkene-enriched reaction mixture;
wherein the first dehydrogenation reaction and the first separation step are conducted in separate reaction and separation units, and the second dehydrogenation reaction and the second separation step are conducted in at least one integrated reaction and separation unit.

2. A method according to claim 1, wherein the alkane feed to the first reactor system comprises steam.

3. A method according to claim 2, wherein the amount of steam added to the first reactor is in a range of from 5 mol% to 60 mol%.

4. A method according to any one of the preceding claims, wherein steam is used as a sweep gas in membrane separation.

5. A method according to any one of the preceding claims, wherein the operating temperature of the first reactor is between 450°C and 550°C.

6. A method according to any one of the preceding claims, wherein the membranes are metal membranes, preferably comprising palladium or a palladium alloy.

7. A method according to claim 6, wherein the membranes are thin palladium membranes, preferably having a thickness of from 1 µm to 3 µm.

8. A method according to any one of the preceding claims, wherein the alkane to be dehydrogenated comprises a hydrocarbon selected from the group consisting of, ethane, propane, butane, ethylbenzene, and mixtures thereof, and preferably is propane.

9. A method according to any one of the preceding claims, wherein unreacted alkane retrieved from the second reactor system is recycled to the first reactor system.

10. A plant for the production of an alkene by the dehydrogenation of a corresponding alkane, said plant comprising a first reactor for conducting a catalytic dehydrogenation reaction, downstream of said first reactor, and in fluid communication therewith, a first membrane separator for separating hydrogen from a dehydrogenation reaction mixture, and downstream of said first membrane separator, and in fluid communication therewith, a second reactor, wherein said first reactor and separator are constructed as separate units, and wherein the second reactor comprises a second membrane separator, said second reactor and separator being constructed as a single unit.

11. A method of modifying an existing olefin production plant comprising at least one membrane-assisted dehydrogenation reactor having a configuration according to closed architecture, by adding to the plant a membrane-assisted dehydrogenation reactor system having a configuration in accordance with open architecture upstream of the at least one existing reactor.

12. A method of modifying an existing olefin production plant comprising at least one membrane-assisted dehydrogenation reactor system having a configuration according to open architecture, comprising a reactor unit and downstream of said reactor unit, a membrane separation unit, the method comprising adding to the plant a membrane-assisted dehydrogenation reactor having a configuration in accordance with closed architecture, downstream of the membrane separation unit of the at least one existing reactor system.

## Patentansprüche

1. Verfahren zur Herstellung eines Alkens durch Dehydrierung eines entsprechenden Alkans, umfassend die Schritte von:
(i) Bereitstellen einer Kohlenwasserstoffquelle, umfassend mindestens ein Alkan;
(ii) Unterziehen, in einem ersten Reaktorsystem, der Kohlenwasserstoffquelle einer ersten Dehydrierungsreaktion in Gegenwart eines Dehydrierungskatalysators, um ein erstes Reaktionsgemisch, umfassend Wasserstoff, nicht reagiertes Alkan und einen Anfangsertrag des Alkens entsprechend dem mindestens einen Alkan, zu bilden;
(iii) in einem ersten Trennungsschritt das Unterziehen des Reaktionsgemisches einer Membrantrennung, um ein Permeat, umfassend Wasserstoff, und ein Retentat, umfassend ein mit Alken angereichertes Reaktionsgemisch zu gewinnen;
(iv) Zuführen des mit Alken angereicherten Reaktionsgemisches einem zweiten Reaktorsystem, wobei nicht reagiertes Alkan, umfasst in dem Reaktionsgemisch, einer zweiten Dehydrierungsreaktion in Gegenwart eines Dehydrierungskatalysators unterzogen wird, um ein zweites Reaktionsgemisch, umfassend Wasserstoff, und einen weiteren Ertrag des Alkens entsprechend dem mindestens einen Alkan zu bilden;
(v) in einem zweiten Trennungsschritt, Unterziehen des zweiten Reaktionsgemisches einer Membrantrennung, um Wasserstoff zu entfernen, um dadurch ein weiteres mit Alken angereichertes Reaktionsgemisch herzustellen;
wobei die erste Dehydrierungsreaktion und der erste Trennungsschritt in getrennten Reaktions- und Trennungseinheiten durchgeführt werden und die zweite Dehydrierungsreaktion und der zweite Trennungsschritt in mindestens einer integrierten Reaktions- und Trennungseinheit durchgeführt werden.

2. Verfahren nach Anspruch 1, wobei die Alkanzufuhr zu dem Reaktorsystem Dampf umfasst.

3. Verfahren nach Anspruch 2, wobei die Menge Dampf, hinzugefügt zu dem ersten Reaktor in einem Bereich von 5 Mol% bis 60 Mol% ist.

4. Verfahren nach einem der vorhergehenden Ansprüche, wobei Dampf als ein Spülgas in der Membrantrennung verwendet wird.

5. Verfahren nach einem der vorhergehenden Ansprüche, wobei die Betriebstemperatur des ersten Reaktors zwischen 450°C und 550°C ist.

6. Verfahren nach einem der vorhergehenden Ansprüche, wobei die Membrane Metallmembrane sind, bevorzugt umfassend Palladium oder eine Palladiumlegierung.

7. Verfahren nach Anspruch 6, wobei die Membranen dünne Palladiummembranen sind, bevorzugt mit einer Dicke von 1 µm bis 3 µm.

8. Verfahren nach einem der vorhergehenden Ansprüche, wobei das zu dehydrierende Alkan einen Kohlenwasserstoff umfasst, ausgewählt aus der Gruppe, bestehend aus Ethan, Propan, Butan, Ethylbenzen und Gemische davon, und bevorzugt Propan ist.

9. Verfahren nach einem der vorhergehenden Ansprüche, wobei nicht reagiertes Alkan, rückgewonnen aus dem zweiten Reaktorsystem, zu dem ersten Reaktorsystem rezykliert wird.

10. Anlage zur Herstellung eines Alkens durch Dehydrierung eines entsprechenden Alkans, die Anlage umfassend einen ersten Reaktor zum Durchführen einer katalytischen Dehydrierungsreaktion stromabwärts von dem ersten Reaktor und in Fluidkommunikation damit, einen ersten Membrantrenner zum Trennen von Wasserstoff von einem Dehydrierungsreaktionsgemisch und stromabwärts von dem ersten Membrantrenner und in Fluidkommunikation damit einen zweiten Reaktor, wobei der erste Reaktor und der Trenner als getrennte Einheiten gebaut sind und wobei der zweite Reaktor einen zweiten Membrantrenner umfasst, wobei der zweite Reaktor und der Trenner als eine einzige Einheit gebaut sind.

11. Verfahren zur Änderung einer bestehenden Olefin-Herstellungsanlage, umfassend mindestens einen membrangestützten Dehydrierungsreaktor mit einer Konfiguration entsprechend geschlossener Architektur durch Hinzufügen zu dem Reaktor eines membrangestützten Dehydrierungsreaktorsystems mit einer Konfiguration entsprechend offener Architektur stromaufwärts von dem mindestens einen bestehenden Reaktor.

12. Verfahren zur Änderung einer bestehenden Olefin-Herstellungsanlage, umfassend mindestens ein membrangestütztes Dehydrierungsreaktorsystem mit einer Konfiguration entsprechend offener Architektur, umfassend eine Reaktoreinheit und stromabwärts von der Reaktoreinheit eine Membrantrennungseinheit, das Verfahren umfassend das Hinzufügen eines membrangestützten Dehydrierungsreaktors zu der Anlage mit einer Konfiguration entsprechend geschlossener Architektur stromabwärts von der Membrantrennungseinheit des mindestens einen bestehenden Reaktorsystems.

## Revendications

1. Procédé pour la production d'un alcène par déshydrogénation d'un alcane correspondant, comprenant les étapes suivantes :
(i) obtention d'une source d'hydrocarbures comprenant au moins un alcane ;
(ii) soumission, dans un premier système de réacteur, de la source d'hydrocarbures à une première réaction de déshydrogénation en présence d'un catalyseur de déshydrogénation, de façon à former un premier mélange réactionnel comprenant de l'hydrogène, un alcane n'ayant pas réagi, et un rendement initial en l'alcène correspondant audit au moins un alcane ;
(iii) dans une première étape de séparation, la soumission du mélange réactionnel à une séparation sur membrane de façon que soit obtenu un perméat comprenant de l'hydrogène et un rétentat comprenant un mélange réactionnel enrichi en alcène ;
(iv) l'introduction dudit mélange réactionnel enrichi en alcène dans un deuxième système de réacteur, dans lequel l'alcane n'ayant pas réagi compris dans ledit mélange réactionnel est soumis à une deuxième réaction de déshydrogénation en présence d'un catalyseur de déshydrogénation de façon à former un deuxième mélange réactionnel comprenant de l'hydrogène et un autre rendement en l'alcène correspondant audit au moins un alcane ;
(v) dans une deuxième étape de séparation, la soumission du deuxième mélange réactionnel à une séparation sur membrane de façon à éliminer l'hydrogène, ce qui produit ainsi un autre mélange réactionnel enrichi en alcène ;
dans lequel la première réaction de déshydrogénation et la première étape de séparation sont effectuées dans des unités de réaction et de séparation séparées, et la deuxième réaction de déshydrogénation et la deuxième étape de séparation sont effectuées dans au moins une unité de réaction et de séparation intégrée.

2. Procédé selon la revendication 1, dans lequel la charge d'alcane pour le premier système de réacteur comprend de la vapeur.

3. Procédé selon la revendication 2, dans lequel la quantité de vapeur ajoutée dans le premier réacteur est située dans la plage allant de 5 % en moles à 60 % en moles.

4. Procédé selon l'une quelconque des revendications précédentes, dans lequel de la vapeur est utilisée en tant que gaz de balayage dans la séparation sur membrane.

5. Procédé selon l'une quelconque des revendications précédentes, dans lequel la température opérationnelle du premier réacteur est comprise entre 450 °C et 550 °C.

6. Procédé selon l'une quelconque des revendications précédentes, dans lequel les membranes sont des membranes métalliques, comprenant de préférence du palladium ou un alliage de palladium.

7. Procédé selon la revendication 6, dans lequel les membranes sont des membranes fines de palladium, ayant de préférence une épaisseur de 1 µm à 3 µm.

8. Procédé selon l'une quelconque des revendications précédentes, dans lequel l'alcane devant être déshydrogéné comprend un hydrocarbure choisi dans le groupe constitué par l'éthane, le propane, le butane, l'éthylbenzène, et leurs mélanges, et de préférence est le propane.

9. Procédé selon l'une quelconque des revendications précédentes, dans lequel l'alcane n'ayant pas réagi récupéré du deuxième système de réacteur est recyclé vers le premier système de réacteur.

10. Installation pour la production d'un alcène par déshydrogénation d'un alcane correspondant, ladite installation comprenant un premier réacteur pour effectuer une réaction de déshydrogénation catalytique, en aval dudit premier réacteur, et en communication de fluide avec celui-ci, un premier séparateur à membrane pour séparer l'hydrogène d'un mélange réactionnel de déshydrogénation, et en aval dudit premier séparateur à membrane, et en communication de fluide avec celui-ci, un deuxième réacteur, dans laquelle lesdits premier réacteur et séparateur sont construits sous la forme d'unités séparées, et dans laquelle le deuxième réacteur comprend un deuxième séparateur à membrane, lesdits deuxième réacteur et séparateur étant construits sous la forme d'une seule unité.

11. Procédé de modification d'une installation de production d'oléfine existante comprenant au moins un réacteur de déshydrogénation assisté par une membrane ayant une configuration conforme à une architecture fermée, par addition à l'installation d'un système de réacteur de déshydrogénation assisté par une membrane ayant une configuration conforme à une architecture ouverte en amont du au moins un réacteur existant.

12. Procédé de modification d'une installation de production d'oléfine existante comprenant au moins un réacteur de déshydrogénation assisté par une membrane ayant une configuration conforme à une architecture ouverte, comprenant une unité de réacteur et, en aval de ladite unité de réacteur, une unité de séparation sur membrane, le procédé comprenant l'addition à l'installation d'un réacteur de déshydrogénation assisté par membrane ayant une configuration conforme à une architecture fermée, en aval de l'unité de séparation sur membrane de du au moins un système de réacteur existant.
